Europäisches Patentamt

European Patent Office

Office européen des brevets

(19)

(11) Veröffentlichungsnummer : **0 362 471 B1**

(12) **EUROPÄISCHE PATENTSCHRIFT**

(45) Veröffentlichungstag der Patentschrift :
**04.03.92 Patentblatt 92/10**

(51) Int. Cl.$^5$ : **A61K 35/10**

(21) Anmeldenummer : **89110360.8**

(22) Anmeldetag : **08.06.89**

(54) **Prophylaktikum für Fische.**

(30) Priorität : **09.09.88 DE 3830616**

(43) Veröffentlichungstag der Anmeldung :
**11.04.90 Patentblatt 90/15**

(45) Bekanntmachung des Hinweises auf die
Patenterteilung :
**04.03.92 Patentblatt 92/10**

(84) Benannte Vertragsstaaten :
**AT BE CH DE ES FR GB GR IT LI LU NL SE**

(56) Entgegenhaltungen :
**EP-A- 0 281 678**
**EP-A- 0 281 679**
**CHEMICAL ABSTRACTS, Band 100, Nr. 10, 5**
**Mai 1984, Seite 334, Zusammenfassung**
**73705b Columbus, Ohio, US S.V. BOL'SHINS-**
**KII et al.: "Method for improving the self-**
**purification in fish-breeding ponds" & GIDRO-**
**BIOL.ZH. 1983, 19(6), 33-5**

(56) Entgegenhaltungen :
**BIOLOGICAL ABSTRACTS, Band 85, Nr. 2,**
**1988, Ref. Nr. 22424 N. J. HUTCHINSON et al.:**
**"Reduced lethality of aluminium, zinc and**
**copper mixtures to American flagfish by com-**
**plexation with humic substances in acidified**
**soft waters" & ENVIRON TOXICOL. CHEM., 6**
**(10), 755-766, 1987**
**BIOLOGICAL ABSTRACTS; Band 77, Nr. 6,**
**1984, Ref. Nr. 42187 P.F. HULSMAN et al.**
**"Mortality of walleye eggs (Stizostedion vi-**
**treum) and rainbow trout (Salmu gairdneri)**
**yolk-sac larvae in low-pH waters of the LaClo-**
**che Mountain area, Osterly (Canada)" &**
**TRANS. AM. FISH SOC. 112(5), 680-688, 1983**
**BIOLOGICAL ABSTRACTS, Band 86, Nr. 7,**
**1988, Ref. Nr. 75722 M.C. BLACK et al.:**
**"Dissolved organic macromolecules reduce**
**the uptake of hydrophobic organic contami-**
**nants by the gills of rainbow trout (Salmo**
**gairdneri)" & ENVIRON. TOXICOL. CHEM.**
**7(7), 593-600, 1988**
**Firmenbroschüre: Stählermatic R Fa. Stähler,**
**6253 Haclamar.**

(73) Patentinhaber : **RÜTGERSWERKE**
**AKTIENGESELLSCHAFT**
**Mainzer Landstrasse 217**
**W-6000 Frankfurt am Main 1 (DE)**

(72) Erfinder : **Riede, Urs N., Prof. Dr.**
**Bifänge 89**
**W-7800 Freiburg-St. Georgen (DE)**
Erfinder : **Spitaler, Ulrich, Dr.**
**Dürkheimer Hohl 2**
**W-6713 Freinsheim (DE)**

EP 0 362 471 B1

## Beschreibung

Die Erfindung betrifft ein neues Mittel zur prophylaktischen Behandlung von Fischen.

Fische, insbesondere importierte Zierfische, werden meist dicht gedrängt in Wasserbehältern transportiert und unterliegen dadurch einem Streß, der verstärkt wird durch einen sich während des Transports erhöhenden Sauerstoffmangel und Ammoniakgehalt des Wassers. Infolgedessen erkranken diese Fische leicht. Insbesondere sinkt ihre Abwehr gegen Pilzbefall so daß während des Transports und in den ersten Tagen nach dem Import viele dieser Fische eingehen.

Durch Zusatz bisher üblicher Prophylaktika zum Wasser wird die durchschnittliche Mortalitätsrate nicht zufriedenstellend gesenkt.

Ähnliche Probleme bestehen bei der gewerblichen Fischzucht. Hier werden vor dem Laichvorgang Milch und Rogen von den Fischen abgestreift und in separaten Laichbecken miteinander vermischt, wobei Laich und die sich entwickelnden Jungfische in großer Dichte vorliegen.

Die bislang üblichen Prophylaktika enthalten darüberhinaus häufig schwach toxische Verbindungen wie Malachitgrün oder Methylblau, die beim Ablassen des Wassers eine Umweltbelastung darstellen.

Es ist daher Aufgabe der Erfindung, ein Prophylaktikum für Fische bereitzustellen, bei dem die Mortalitätsrate gestreßter Fische stark vermindert ist und die keine toxischen oder umweltbelastenden Stoffe enthalten.

Die Aufgabe wird gelöst durch die Verwendung von Mitteln, die niedermolekulare Alkalihuminate enthalten, gemäß der Ansprüche 1 bis 5.

Es wurde gefunden, daß die Widerstandsfähigkeit gestreßter Fische und damit Ihre Überlebensquote enorm steigt, wenn dem sie umgebenden Wasser ein Mittel zugegeben wird, das im wesentlichen niedermolekulare Alkalihuminate enthält. Insbesondere wird die Anfälligkeit gegen Pilzerkrankungen stark reduziert.

Dies ist insofern überraschend, als handelsübliche Präparate, die Huminsäure enthalten, keinen derartigen Effekt zeigen.

Natürliche und synthetische niedermolekulare Alkalihuminate und Verfahren zu ihrer Herstellung sind in früheren Anmeldungen der Anmelderin (EP-A-0 281 679, EP-A-0 281 678 und EP-A-0 313 718) beschrieben. Von daher war bekannt, daß diese niedermolekularen Alkalihuminate als Heilmittel in der Wundheilung wirksam sind.

Ihre Wirksamkeit und Verwendung als Prophylaktikum für Fische, insbesondere gegen Pilzerkrankungen, ist auch von daher neu und überraschend. Niedermolekulare Alkalihuminate sind wasserlösliche Substanzen, wobei sich die Substanzen in der wäßrigen Lösung nicht verändern. Daher ist die einfachste Applikationsform eine wäßrige Lösung. Diese wäßrige Lösung kann außer der wesentlichen Komponente der niedermolekularen Alkalihuminate noch weitere in der Aquaristik übliche Zusatzstoffe wie Vitamine, Spurenelemente oder Schutzkolloide enthalten.

Die prophylaktische Behandlung der Fische erfolgt am einfachsten in der Weise, daß dem Wasser, in dem die Fische während des Transports oder danach leben, eine gewisse Menge des erfindungsgemäßen Prophylaktikums zugesetzt wird. Dies kann bei Zierfischen im Aquarium und bei Nutzfischen in den Zuchtteichen erfolgen. Desweiteren kann dem Wasser des im separaten Becken zur Aufzucht aufbewahrten Laichs oder der gerade geschlüpften Zierfische erfindungsgemäßes Prophylaktikum zugegeben werden. Die Konzentration der niedermolekularen Alkalihuminate liegt dabei je nach Härtegrad des Wassers im Bereich von 0,5 bis 10 ppm.

Bei weichem Wasser, oder bei Mitverwendung entionisierender Stoffe ist eine Konzentration von 0,8 bis 2 ppm ausreichend. Es hat sich als günstig erwiesen, das Prophylaktikum in 3 bis 5tägigem Abstand nachzudosieren.

Eine andere Behandlungsmethode besteht darin, daß die gestreßten Fische, etwa nach einem Transport, kurzzeitig (10 bis 60 min) in ein Bad eingebracht werden, das das Prophylaktikum enthält. Dabei sollte die Konzentration der niedermolekularen Alkalihuminate im Bad höher sein als bei der Langzeitbehandlung. Im allgemeinen reicht eine Konzentration von 2 bis 20 ppm niedermolekularer Alkalihuminate in einem derartigen Bad.

Die Wirkung des erfindungsgemäßen Prophylaktikums ist bereits nach kurzer Zeit festzustellen. Die Fische werden lebhaft und der typische Glanz eines gesunden Fisches stellt sich ein. Die Überlebensrate steigt signifikant. Während bei manchen Fischsendungen, auch bei Verwendung handelsüblicher Wasseraufbereiter oder Prophylaktika, 30 bis 40 % der Fische innerhalb der ersten 8 Tage nach Ankunft eingehen, werden bei Verwendung des erfindungsgemäßen Prophylaktikums Überlebensquoten von 90 bis 99 % erreicht.

## Beispiele

Jeweils 170 Fische verschiedener Arten wurden nach einem Transport in drei verschiedene Becken mit je 70 l Wasser eingebracht und im Verlauf von 7 Tagen vergleichend beobachtet. Die Haltungs- und Fütterungsbedingungen waren dabei jeweils gleich. Lediglich das im Prinzip gleiche Wasser war durch Zusätze verändert:

Becken a: kein Zusatz
Becken b: 20 ml (vorgeschriebene Dosis) eines handelsüblichen, huminsäurehaltigen Prophylaktikums
Becken c: 3 ml einer 2%igen wäßrigen Lösung eines niedermolekularen Alkalihuminats, hergestellt gemäß
EP-A-0 313 718. Nach 4 Tagen wurden weitere 2 ml der 2%igen Lösung nachdosiert.
Die Ergebnisse nach 7 Tagen finden sich in der folgenden Aufstellung:

|  | Becken a | Becken b | Becken c |
|---|---|---|---|
| Fischart: |  |  |  |
| Schwertträger, gold |  |  |  |
| Anzahl gestorbener Tiere | 25 | 19 | 0 |
| Mortalitätsrate (%) | 14,7 | 11,2 | 0 |
| Vitalität der lebenden Tiere | gut | gut | sehr gut |
| Pilzbefall | schwach | schwach | kein |

| | Becken a | Becken b | Becken c |
|---|---|---|---|
| **Fischart:** | | | |
| Platy, rot | | | |
| Anzahl gestorbener Tiere | 20 | 6 | 1 |
| Mortalitätsrate (%) | 11,7 | 3,5 | 0,6 |
| Vitalität der lebenden Tiere | mäßig | gut | gut |
| Pilzbefall | schwach | schwach | kein |
| | | | |
| **Fischart:** | | | |
| Roter Phantomsalmler | | | |
| Anzahl gestorbener Tiere | 86 | 32 | 10 |
| Mortalitätsrate (%) | 50,5 | 18,8 | 5,7 |
| Vitalität der lebenden Tiere | schlecht | mäßig | mäßig |
| Pilzbefall | stark | schwach | schwach |
| | | | |
| **Fischart:** | | | |
| Guppy, Weibchen | | | |
| Anzahl gestorbener Tiere | 31 | 18 | 9 |
| Mortalitätsrate (%) | 18,2 | 10,5 | 5,1 |
| Vitalität der lebenden Tiere | mäßig | mäßig | gut |
| Pilzbefall | schwach | schwach | kein |

**Patentansprüche**

1. Verwendung niedermolekularer Alkalihuminate zur Herstellung eines Antistressmittels zur prophylaktischen Behandlung von Fischen.

2. Verwendung des nach Anspruch 1 hergestellten Mittels zur prophylaktischen Behandlung von Fischen, **dadurch gekennzeichnet**, daß es dem Wasser, in dem die Fische leben, zugesetzt wird.

3. Verwendung des nach Anspruch 1 hergestellten Mittels zur prophylaktischen Behandlung von Fischen, **dadurch gekennzeichnet**, daß es dem Laich zugesetzt wird.

4. Verwendung nach den Ansprüchen 2 und 3, **dadurch gekennzeichnet**, daß dem Wasser soviel Prophylaktikum zugesetzt wird, daß die Konzentration von niedermolekularen Alkalihuminaten im Bereich von 0,5 bis 10 ppm liegt.

5. Verwendung nach den Ansprüchen 2 und 3, dadurch gekennzeichnet, daß die Konzentration von niedermolekularen Alkalihuminaten im Bereich von 0,8 - 2 ppm liegt.

**Claims**

1. Use of low-molecular alkali huminates for the preparation of an antistress agent for the prophylactic treatment of fish.

2. Use of the agent prepared according to Claim 1 for the prophylactic treatment of fish, characterized in that it is added to the water in which the fish live.

3 Use of the agent prepared according to Claim 1 for the prophylactic treatment of fish, characterized in that it is added to the spawn.

4. Use according to Claims 2 and 3, characterized in that sufficient prophylactic is added to the water that the concentration of low-molecular alkali huminates is in the range of from 0.5 to 10 ppm.

5. Use according to Claims 2 and 3, characterized in that the concentration of low-molecular alkali huminates is in the range of from 0.8 to 2 ppm.

**Revendications**

1. Utilisation d'humates alcalins de bas poids moléculaire pour la préparation d'un agent anti-stress destiné au traitement prophylactique des poissons.

2. Utilisation de l'agent préparé selon la revendication 1 pour le traitement prophylactique des poissons, caractérisée en ce qu'il est ajouté à l'eau dans laquelle vivent les poissons.

3. Utilisation de l'agent préparé selon la revendication 1 pour le traitement prophylactique des poissons, caractérisée en ce qu'il est ajouté au frai.

4. Utilisation selon les revendications 2 et 3, caractérisée en ce qu'on ajoute à l'eau une quantité d'agent prophylactique telle que la concentration d'humates alcalins de bas poids moléculaire se situe dans la plage de 0,5 à 10 ppm.

5. Utilisation selon les revendications 2 et 3, caractérisée en ce que la concentration d'humates alcalins de bas poids moléculaire se situe dans la plage de 0,8 à 2 ppm.